# FASCICULE DE BREVET EUROPEEN

(11) **EP 0 697 211 B1**
(45) Date de publication et mention de la délivrance du brevet: **09.10.1996**
(21) Numéro de dépôt: 95401567.3
(22) Date de dépôt: 29.06.1995
(51) Int. Cl.: A61K 7/13

(54) **Utilisation d'un sel métallique dans un procédé de teinture en deux temps mettant en oeuvre un composé indolique**
Verwendung eines Metallsalzes in einem zweistufigen Färbeverfahren mit einer Indolverbindung
Use of a metallic salt in a two-steps dyeing process based on a indole compound

(30) Priorité: 22.07.1994 FR 9409116
(43) Date de publication de la demande: 21.02.1996
(73) Titulaire: L'OREAL, F-75008 Paris (FR)
(72) Inventeur: Samain, Henri, F-91570 Bievres (FR)
(74) Mandataire: Andral, Christophe André Louis

(56) Documents cités:
- EP-A- 0 335 477
- EP-A- 0 342 034
- EP-A- 0 621 029
- WO-A-93/18738
- FR-A- 1 133 594
- GB-A- 2 187 456

## Description

La présente invention a pour objet l'utilisation d'un sel métallique dans un procédé de teinture des fibres kératiniques en deux temps mettant en oeuvre un composé indolique.

La coloration des cheveux, de la peau et des poils, en particulier d'origine humaine, provient principalement des pigments mélaniques sécrétés par les mélanocytes. Ces pigments d'origine naturelle, comprennent en particulier des eumélanines. Leur biosynthèse naturelle s'effectue en plusieurs étapes par polymérisation des produits d'oxydation d'un acide aminé : la tyrosine. L'un des produits d'oxydation de la tyrosine est le 5,6-dihydroxyindole qui polymérise à son tour en eumélanines.

On a déjà proposé dans le passé de repigmenter les cheveux humains qui ont tendance à blanchir ou à grisonner avec du 5,6-dihydroxyindole. Le 5,6-dihydroxyindole est généralement utilisé dans des compositions aqueuses avec lesquelles il est possible de teindre les fibres kératiniques et en particulier les cheveux, cette teinture pouvant être effectuée en plusieurs fois ou de façon progressive sans autre agent oxydant que l'oxygène de l'air. Il est possible de cette façon de repigmenter les cheveux en partant de nuances relativement claires par une application du produit, jusque dans des nuances de plus en plus soutenues, par superposition des applications.

On recherche, lors de la coloration des fibres kératiniques et plus particulièrement des cheveux, avec le 5,6-dihydroxyindole ou ses dérivés, l'obtention de nuances plus ou moins sombres et neutres qui n'évoluent pas au cours du temps, notamment lors de l'exposition à la lumière, à la transpiration ou à des lavages répétés.

Il faut noter, en particulier, que les nuances de gris peu soutenu ont tendance à évoluer au cours du temps de façon plus rapide et plus marquée que les nuances sombres.

Dans la demande de brevet FR-A-2 700 266, il a été proposé l'utilisation d'un sel de magnésium dans un procédé de teinture des cheveux mettant en oeuvre le 5,6-dihydroxyindole ou un de ses dérivés pour améliorer la ténacité des colorations vis à vis de la lumière. Cependant l'utilisation des sels de magnésium n'est pas entièrement satisfaisante notamment en ce qui concerne les nuances gris clair qui ont malgré cela tendance à évoluer au cours du temps.

C'est afin de résoudre ce problème que la demanderesse a découvert ce qui fait l'objet de l'invention.

L'invention a donc pour objet l'utilisation d'un sel métallique, dans un procédé de teinture en deux temps des fibres kératiniques et en particulier des fibres kératiniques humaines telles que les cheveux mettant en oeuvre, dans un premier temps, une composition (A) contenant dans un milieu approprié pour la teinture au moins un composé indolique de formule (I) suivante : 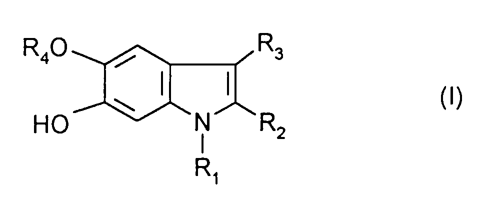
dans laquelle :
R₁, R₂, R₃, R₄; identiques ou différents, désignent un atome d'hydrogène ou un radical alkyle en C₁-C₄, R₁, R₂, R₃ désignant hydrogène lorsque R₄ désigne un radical alkyle en C₁-C₄, et dans un deuxième temps, une composition (B) contenant dans un milieu approprié pour la teinture au moins un sel métallique choisi parmi les composés présentant une activité catalytique d'oxydation par l'air et dont le potentiel d'oxydo-réduction normal E₀ est inférieur à -0,25V, afin d'obtenir des colorations dans des nuances grises n'évoluant pas au cours du temps.

La demanderesse a remarqué en particulier que les colorations obtenues sont particulièrement résistantes à la lumière et ceci de façon supérieure à l'état de la technique, c'est à dire que la lumière n'entraîne pas une dégradation importante au cours du temps des colorations obtenues. Ces colorations présentent également de bonnes résistances aux lavages répétés et à la transpiration.

Dans la formule (I) ci-dessus, le radical alkyle désigne de préférence méthyle ou éthyle et R₁, R₂ et R₃ ne désignent pas simultanément un radical alkyle en C₁-C₄.

Les composés indoliques de formule (I) préférés sont choisis parmi le 5,6-dihydroxyindole, le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole. Un composé indolique de formule (I) particulièrement préféré est le 5,6-dihydroxyindole.

Le ou les sels métalliques particuliers de l'invention peuvent être utilisés sous forme solubilisée ou dispersée et sont plus particulièrement choisis parmi les sels de manganèse, de zinc, de fer et / ou de cobalt. Ces sels peuvent être utilisés seuls ou en mélange.

Parmi ces composés, on peut citer les acétates, les halogénures, les sulfates et les carbonates de manganèse, de zinc, de fer et / ou de cobalt ainsi que leurs hydrates.

De tels composés sont par exemple le diacétate de manganèse tétrahydrate, le dichlorure de manganèse et ses hydrates, le dihydrogénocarbonate de manganèse, I'acétylacétonate de manganèse, le triacétate de manganèse et ses hydrates, le trichlorure de manganèse, le dichlorure de zinc, le diacétate de zinc dihydrate, le carbonate de zinc, le dinitrate de zinc, le sulfate de zinc, le dichlorure de fer, le sulfate de fer, le diacétate de fer, le diacétate de cobalt tétrahydrate, le carbonate de cobalt, le dichlorure de cobalt, le dinitrate de cobalt, le sulfate de cobalt heptahydrate.

Le diacétate de manganèse tétrahydrate et le dichlorure de zinc sont particulièrement préférés.

Le ou les sels métalliques utilisés selon l'invention sont généralement mis en oeuvre dans des proportions comprises entre 0,001 et 4 % en poids d'équivalent métal par rapport au poids total de la composition (B) et de préférence entre 0,005 et 2 % en poids d'équivalent métal par rapport au poids total de la composition (B).

L'application des compositions (A) et (B) est de préférence séparée par une étape de rinçage.

La composition (A) contenant au moins un composé indolique de formule (I) est appliquée sur les fibres kératiniques et en particulier les fibres kératiniques humaines telles que les cheveux avec un temps de pose généralement compris entre 15 secondes et 30 minutes.

La composition (B) contenant le ou les sels métalliques de l'invention est appliquée sur les fibres kératiniques et en particulier les fibres kératiniques humaines telles que les cheveux avec un temps de pose généralement compris entre 15 secondes et 30 minutes.

Le procédé de teinture utilisant au moins un sel métallique de l'invention est mis en oeuvre en n'utilisant pas d'autre oxydant que l'oxygène de l'air.

Le ou les composés indoliques de formule (I) utilisés selon l'invention, sont généralement mis en oeuvre dans des proportions suffisantes pour repigmenter les fibres kératiniques et en particulier les cheveux. Ces proportions sont de préférence comprises entre 0,1 et 5 % en poids et encore plus préférentiellement entre 0,2 et 3 % en poids par rapport au poids total de la composition (A).

Les compositions (A) et (B) sont de préférence aqueuses ou constituées d'un mélange d'eau et d'un solvant cosmétiquement acceptable.

Lorsque ces compositions contiennent un solvant cosmétiquement acceptable, celui-ci peut être choisi parmi les alcools inférieurs en C₁-C₄ tels que l'éthanol, l'alcool propylique, l'alcool isopropylique, l'alcool tertiobutylique ; les glycols tels que l'éthylèneglycol, le propylèneglycol ; les éthers de glycols tels que les éthers monométhylique, monoéthylique ou monobutylique de l'éthylèneglycol, les monométhyléthers du propylèneglycol et du dipropylèneglycol ; les esters inférieurs comme le lactate de méthyle et l'acétate de monoéthyléther de l'éthylèneglycol.

Les solvants particulièrement préférés sont choisis parmi l'éthanol et le propylèneglycol.

Lorsqu'ils sont présents, les solvants représentent de préférence de 0,5 à 50 % en poids et de préférence de 2 à 20 % en poids par rapport au poids total de la composition (A) ou (B).

La composition (A) utilisée selon l'invention a généralement un pH compris entre 5 et 10 et de préférence entre 7 et 9,5. Ce pH est ajusté à l'aide d'agents alcalinisants ou acidifiants classiquement utilisés dans le domaine de la teinture des cheveux.

Parmi les agents alcalinisants, on peut citer à titre d'exemple, l'ammoniaque, les alcanolamines telles que par exemple les mono-, di- et triéthanolamines et leurs dérivés, les hydroxydes de sodium ou de potassium tels que par exemple la soude et la potasse, les alkylamines, les carbonates alcalins ou d'ammonium.

Parmi les agents acidifiants, on peut citer à titre d'exemple, l'acide orthophosphorique, l'acide lactique, l'acide acétique, l'acide tartrique, l'acide chlorhydrique et l'acide citrique.

La composition (B) utilisée selon l'invention a généralement un pH compris entre 5 et 11 et de préférence entre 7 et 10. Ce pH est ajusté à l'aide d'agents alcalinisants ou acidifiants classiquement utilisés dans le domaine de la teinture des cheveux et tels que décrits précédemment.

Les compositions (A) et / ou (B) mises en oeuvre selon l'invention peuvent se présenter sous la forme de lotions plus ou moins épaissies, de gels, de shampooings, d'émulsions telles que des laits et des crèmes ou elles peuvent être propulsées sous forme de mousses aérosols.

Selon une forme de réalisation particulièrement avantageuse et préférée de l'invention, la composition (A) se présente sous la forme d'un gel et la composition (B) sous la forme d'un shampooing.

Dans cette forme de réalisation particulière, la composition (A) sous forme de gel et renfermant le ou les composés indoliques de formule (I) est appliquée sur les fibres kératiniques avec un temps de pose variant entre 15 secondes et 30 minutes puis après rinçage à l'eau, la composition (B) sous forme de shampooing et renfermant le ou les sels métalliques utilisés selon l'invention est émulsionnée sur les fibres pendant quelques secondes à quelques minutes. Les fibres sont ensuite rincées et séchées.

Les compositions (A) et / ou (B) peuvent contenir en outre au moins un adjuvant choisi parmi les adjuvants utilisés classiquement dans les compositions pour la teinture des cheveux tels que les agents tensio-actifs anioniques, cationiques, nonioniques, amphotères, zwittérioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les agents conservateurs, les agents opacifiants, les filtres solaires, etc...

Les exemples suivants sont destinés à illustrer l'invention sans pour autant en limiter la portée.

### EXEMPLES

### EXEMPLE 1 COMPARATIF

On prépare la composition A suivante :

| | |
|---|---|
| - 5,6-dihydroxyindole | 0,5 g |
| - Gomme de xanthane | 1,8 g |
| - Alkylpolyglucoside en solution aqueuse à 60 % de matière active (M.A.) vendu par la société SEPPIC | 4 g |
| - Acide tartrique | 0,35 g |
| - Ethanol | 12 g |
| - Triéthanolamine q.s. | pH 8,5 |
| - Eau déminéralisée q.s.p. | 100 g |

On prépare la composition (B₁) suivante, qui est un shampooing ne faisant pas partie de l'invention :

| | |
|---|---|
| - Monoisopropanolamide d'acide de coprah vendu par la société ALLBRIGHT ET WILSON SAINT MIHIEL | 2,5 g |
| - Lauryléthersulfate de monoéthanolamine en solution aqueuse à 28 % de M.A. | 40 g |
| - Cocoylbétaïne en solution aqueuse à 32 % de M.A. vendue par la société HENKEL | 10 g |
| - Monosulfosuccinate de monoéthanolamide ricinoléïque, sel disodique vendu par la société WITCO | 2 g |
| - Hydroxyde de sodium q.s.p. | pH 9,5 |
| - Parfum q.s. | |
| - Eau déminéralisée q.s.p. | 100 g |

On prépare la composition (B₂) suivante, qui est un shampooing faisant partie de l'invention :

| | |
|---|---|
| - Monoisopropanolamide d'acide de coprah vendu par la société ALLBRIGHT ET WILSON SAINT MIHIEL | 2,5 g |
| - Lauryléthersulfate de monoéthanolamine en solution aqueuse à 28 % de M.A. | 40 g |
| - **Diacétate de manganèse, tétrahydrate** | **1 g** |
| - Cocoylbétaïne en solution aqueuse à 32 % de M.A. vendue par la société HENKEL | 10 g |
| - Monosulfosuccinate de monoéthanolamide ricinoléïque, sel disodique vendu par la société WITCO | 2 g |
| - Hydroxyde de sodium q.s.p. | pH 9,5 |
| - Parfum q.s. | |
| - Eau déminéralisée q.s.p. | 100 g |

On applique la composition (A) sur deux mèches de cheveux gris naturels à 90 % de blancs pendant 10 minutes puis on les rince à l'eau.
Sur la première mèche, on applique ensuite la composition (B₁) que l'on émulsionne pendant environ 30 secondes puis on rince à l'eau.
Sur la deuxième mèche, on applique ensuite la composition (B₂) que l'on émulsionne pendant environ 30 secondes puis on rince à l'eau.
Les deux mèches sont ensuite séchées.

Les deux mèches présentent une coloration identique gris métallique.

Les deux mèches sont ensuite laissées à l'air libre et à la lumière naturelle pendant une période de trois semaines.
On observe alors que la mèche ayant subi le shampooing avec la composition (B₁) ne faisant pas partie de l'invention présente une coloration qui est devenue gris doré alors que la mèche ayant subi le shampooing avec la composition (B₂) faisant partie de l'invention présente toujours une coloration gris métallique.

### EXEMPLE 2 COMPARATIF

On prépare la composition (B₃) suivante, qui est un shampooing faisant partie de l'invention :

| | |
|---|---|
| - Monoisopropanolamide d'acide de coprah vendu par la société ALLBRIGHT ET WILSON SAINT MIHIEL | 2,5 g |
| - Lauryléthersulfate de monoéthanolamine en solution aqueuse à 28 % de M.A. | 40 g |
| - **Dichlorure de zinc** | **1 g** |
| - Cocoylbétaïne en solution aqueuse à 32 % de M.A. vendue par la société HENKEL | 10 g |
| - Monosulfosuccinate de monoéthanolamide ricinoléïque, sel disodique vendu par la société WITCO | 2 g |
| - Hydroxyde de sodium q.s.p. | pH 9,5 |
| - Parfum q.s. | |
| - Eau déminéralisée q.s.p. | 100 g |

On applique la composition (A) telle que décrite à l'exemple 1 ci-dessus sur deux mèches de cheveux gris naturels à 90 % de blancs pendant 10 minutes puis on les rince à l'eau.
Sur la première mèche, on applique ensuite la composition (B₁) telle que décrite ci-dessus à l'exemple 1 que l'on émulsionne pendant environ 30 secondes puis on rince à l'eau.
Sur la deuxième mèche, on applique ensuite la composition (B₃) que l'on émulsionne pendant environ 30 secondes puis on rince à l'eau.
Les deux mèches sont ensuite séchées.

Les deux mèches subissent alors une nouvelle fois le traitement décrit précédemment puis elles sont laissées à l'air libre et à la lumière naturelle pendant une période de trois semaines.

L'évaluation des colorations obtenues juste après la deuxième application (t = 0) et leur évolution au bout de trois semaines (t = 3 semaines)a été réalisée dans le système Lab et figure dans le tableau ci-après :

| **Composition** | **Coloration à t = 0** | | | **Coloration à t = 3 semaines** | | | **Ecart** | | |
|---|---|---|---|---|---|---|---|---|---|
| | **L** | **a** | **b** | **L** | **a** | **b** | **L** | **a** | **b** |
| **B**_{**1**} | 34 | 1,8 | 3,4 | 32 | 2,3 | 5,2 | -2 | 0,5 | 1,7 |
| **B**_{**3**} | 30 | 1,2 | 1,3 | 29,6 | 1,2 | 3 | -0,4 | 0 | 1,7 |

Selon le système Lab, L indique la clarté. Plus la valeur de L est élevée, plus la couleur est claire. Inversement, plus la valeur de L est faible, plus la couleur est foncée et donc puissante.

La teinte et la saturation sont exprimées par a et b.
a et b indiquent deux axes de couleur, a l'axe rouge vert et b l'axe jaune bleu.

Une valeur positive de a correspond à une teinte rouge d'autant plus saturée que la valeur absolue de a est élevée.

Une valeur négative de a correspond à une teinte verte d'autant plus saturée que la valeur absolue de a est élevée.

Une valeur positive de b correspond à une teinte jaune d'autant plus saturée que la valeur absolue de b est élevée.

Une valeur négative de b correspond à une teinte bleue d'autant plus saturée que la valeur absolue de b est élevée.

Des valeurs proches de zéro pour a ou b correspondent à des teintes grises.

Au bout de trois semaines, les mèches teintes avec la composition B₃ faisant partie de l'invention présentent une couleur ayant moins évolué que celle des mèches teintes avec la composition B₁ ne faisant pas partie de l'invention.

## Revendications

1. Utilisation d'un sel métallique, dans un procédé de teinture en deux temps des fibres kératiniques et en particulier des fibres kératiniques humaines telles que les cheveux mettant en oeuvre, dans un premier temps, une composition (A) contenant dans un milieu approprié pour la teinture au moins un composé indolique de formule (I) suivante : 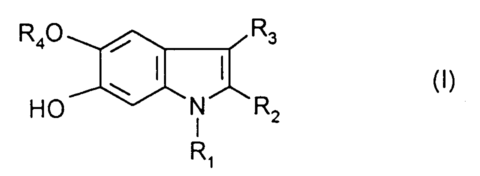 dans laquelle :
R₁, R₂, R₃, R₄; identiques ou différents, désignent un atome d'hydrogène ou un radical alkyle en C₁-C₄, R₁, R₂, R₃ désignant hydrogène lorsque R₄ désigne un radical alkyle en C₁-C₄, et dans un deuxième temps, une composition (B) contenant dans un milieu approprié pour la teinture au moins un sel métallique choisi parmi les composés présentant une activité catalytique d'oxydation par l'air et dont le potentiel d'oxydo-réduction normal E₀ est inférieur à -0,25V, afin d'obtenir des colorations dans des nuances grises n'évoluant pas au cours du temps.

2. Utilisation selon la revendication 1, caractérisée par le fait que dans la formule (I), le radical alkyle désigne méthyle ou éthyle et R₁, R₂ et R₃ ne désignent pas simultanément un radical alkyle en C₁-C₄.

3. Utilisation selon l'une quelconque des revendications 1 ou 2, caractérisée par le fait que le ou les composés indoliques de formule (I) sont choisis parmi le 5,6-dihydroxyindole, le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole.

4. Utilisation selon la revendication 3, caractérisée par le fait que le composé indolique de formule (I) est le 5,6-dihydroxyindole.

5. Utilisation selon l'une quelconque des revendications précédentes, caractérisée par le fait que le ou les sels métalliques sont utilisés sous forme solubilisée ou dispersée et sont choisis parmi les sels de manganèse, de zinc, de fer et / ou de cobalt et leurs mélanges.

6. Utilisation selon la revendication 5, caractérisée par le fait que le ou les sels métalliques sont choisis parmi les acétates, les halogénures, les sulfates et les carbonates de manganèse, de zinc, de fer et / ou de cobalt ainsi que leurs hydrates.

7. Utilisation selon la revendication 6, caractérisée par le fait que le ou les sels métalliques sont choisis parmi le diacétate de manganèse tétrahydrate, le dichlorure de manganèse et ses hydrates, le dihydrogénocarbonate de manganèse, I'acétylacétonate de manganèse, le triacétate de manganèse et ses hydrates, le trichlorure de manganèse, le dichlorure de zinc, le diacétate de zinc dihydrate, le carbonate de zinc, le dinitrate de zinc, le sulfate de zinc, le dichlorure de fer, le sulfate de fer, le diacétate de fer, le diacétate de cobalt tétrahydrate, le carbonate de cobalt, le dichlorure de cobalt, le dinitrate de cobalt, le sulfate de cobalt heptahydrate.

8. Utilisation selon la revendication 7, caractérisée par le fait que le ou les sels métalliques sont choisis parmi le diacétate de manganèse tétrahydrate et le dichlorure de zinc.

9. Utilisation selon l'une quelconque des revendications précédentes, caractérisée par le fait que le ou les sels métalliques sont mis en oeuvre dans des proportions comprises entre 0,001 et 4 % en poids d'équivalent métal par rapport au poids total de la composition (B).

10. Utilisation selon la revendication 9, caractérisée par le fait que le ou les sels métalliques sont mis en oeuvre dans des proportions comprises entre 0,005 et 2 % en poids d'équivalent métal par rapport au poids total de la composition (B).

11. Utilisation selon l'une quelconque des revendications précédentes, caractérisée par le fait que la composition (A) est appliquée sur les fibres kératiniques et en particulier les fibres kératiniques humaines telles que les cheveux avec un temps de pose compris entre 15 secondes et 30 minutes.

12. Utilisation selon l'une quelconque des revendications précédentes, caractérisée par le fait que la composition (B) est appliquée sur les fibres kératiniques et en particulier les fibres kératiniques humaines telles que les cheveux avec un temps de pose compris entre 15 secondes et 30 minutes.

13. Utilisation selon l'une quelconque des revendications précédentes, caractérisée par le fait que l'application des compositions (A) et (B) est séparée par une étape de rinçage.

14. Utilisation selon l'une quelconque des revendications précédentes, caractérisée par le fait que le ou les composés indoliques de formule (I) sont mis en oeuvre dans des proportions suffisantes pour repigmenter les fibres kératiniques.

15. Utilisation selon la revendication 14, caractérisée par le fait que le ou les composés indoliques de formule (I) sont mis en oeuvre dans des proportions comprises 0,1 et 5 % en poids par rapport au poids total de la composition (A).

16. Utilisation selon la revendication 15, caractérisée par le fait que le ou les composés indoliques de formule (I) sont mis en oeuvre dans des proportions comprises 0,2 et 3 % en poids par rapport au poids total de la composition (A).

17. Utilisation selon l'une quelconque des revendications précédentes, caractérisée par le fait que les compositions (A) et (B) sont aqueuses ou constituées d'un mélange d'eau et d'un solvant cosmétiquement acceptable.

18. Utilisation selon la revendication 17, caractérisée par le fait que le solvant cosmétiquement acceptable est choisi parmi les alcools inférieurs en C₁-C₄, les glycols, les éthers de glycols, les esters inférieurs.

19. Utilisation selon l'une quelconque des revendications précédentes, caractérisée par le fait que la composition (A) a un pH compris entre 5 et 10.

20. Utilisation selon la revendication 19, caractérisée par le fait que la composition (A) a un pH compris entre 7 et 9,5.

21. Utilisation selon l'une quelconque des revendications précédentes, caractérisée par le fait que la composition (B) a un pH compris entre 5 et 11.

22. Utilisation selon la revendication 21, caractérisée par le fait que la composition (B) a un pH compris entre 7 et 10.

23. Utilisation selon l'une quelconque des revendications précédentes, caractérisée par le fait que les compositions (A) et / ou (B) se présentent sous la forme de lotions plus ou moins épaissies, de gels, de shampooings, d'émulsions telles que des laits et des crèmes ou mousses.

24. Utilisation selon la revendication 23, caractérisée par le fait que la composition (A) se présente sous la forme d'un gel et la composition (B) sous la forme d'un shampooing.

25. Utilisation selon l'une quelconque des revendications précédentes, caractérisée par le fait que les compositions (A) et / ou (B) peuvent contenir en outre au moins un adjuvant choisi parmi les agents tensio-actifs anioniques, cationiques, nonioniques, amphotères, zwittérioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les agents conservateurs, les agents opacifiants, les filtres solaires.

## Claims

1. Use of a metallic salt, in a two-stage process for dyeing keratinous fibres and in particular human keratinous fibres such as the hair using, in a first stage, a composition (A) containing, in an appropriate medium for dyeing, at least one indole compound of the following formula (I): 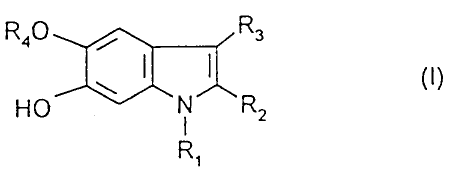 in which:
R₁, R₂, R₃, R₄, which are identical or different, designate a hydrogen atom or a C₁-C₄ alkyl radical, R₁, R₂, R₃ designating hydrogen when R₄ designates a C₁-C₄ alkyl radical, and in a second stage, a composition (B) containing, in an appropriate medium for dyeing, at least one metallic salt chosen from the compounds having a catalytic activity for oxidation by air and whose normal oxidation-reduction potential E₀ is less than -0.25 V, in order to obtain colours in grey shades which do not vary over time.

2. Use according to Claim 1, characterized in that in the formula (I), the alkyl radical designates methyl or ethyl and R₁, R₂ and R₃ do not simultaneously designate a C₁-C₄ alkyl radical.

3. Use according to either of Claims 1 and 2, characterized in that the indole compound(s) of formula (I) are chosen from 5,6-dihydroxyindole, 2-methyl-5,6-dihydroxyindole, 3-methyl 5,6-dihydroxyindole, 1-methyl-5,6-dihydroxyindole, 2,3-dimethyl-5,6-dihydroxyindole, 5-methoxy-6-hydroxyindole.

4. Use according to Claim 3, characterized in that the indole compound of formula (I) is 5,6-dihydroxy-indole.

5. Use according to any one of the preceding claims, characterized in that the metallic salt(s) are used in solubilized or dispersed form and are chosen from manganese, zinc, iron and/or cobalt salts and mixtures thereof.

6. Use according to Claim 5, characterized in that the metallic salt(s) are chosen from manganese, zinc, iron and/or cobalt acetates, halides, sulphates and carbonates as well as hydrates thereof.

7. Use according to Claim 6, characterized in that the metallic salt(s) are chosen from manganese diacetate tetrahydrate, manganese dichloride and hydrates thereof, manganese dihydrogen carbonate, manganese acetyl acetonate, manganese triacetate and hydrates thereof, manganese trichloride, zinc dichloride, zinc diacetate dihydrate, zinc carbonate, zinc dinitrate, zinc sulphate, iron chloride, iron sulphate, iron diacetate, cobalt diacetate tetrahydrate, cobalt carbonate, cobalt dichloride, cobalt dinitrate, cobalt sulphate heptahydrate.

8. Use according to Claim 7, characterized in that the metallic salt(s) are chosen from manganese diacetate tetrahydrate and zinc dichloride.

9. Use according to any one of the preceding claims, characterized in that the metallic salt(s) are used in proportions of between 0.001 and 4% by equivalent metal weight relative to the total weight of the composition (B).

10. Use according to Claim 9, characterized in that the metallic salt(s) are used in proportions of between 0.005 and 2% by equivalent metal weight relative to the total weight of the composition (B).

11. Use according to any one of the preceding claims, characterized in that the composition (A) is applied to the keratinous fibres and in particular the human keratinous fibres such as hair with an exposure time of between 15 seconds and 30 minutes.

12. Use according to any one of the preceding claims, characterized in that the composition (B) is applied to the keratinous fibres and in particular the human keratinous fibres such as hair with an exposure time of between 15 seconds and 30 minutes.

13. Use according to any one of the preceding claims, characterized in that the application of the compositions (A) and (B) is separated by a rinsing stage.

14. Use according to any one of the preceding claims, characterized in that the indole compound(s) of formula (I) are used in proportions sufficient to repigment keratinous fibres.

15. Use according to Claim 14, characterized in that the indole compound(s) of formula (I) are used in proportions of between 0.1 and 5% by weight relative to the total weight of the composition (A).

16. Use according to Claim 15, characterized in that the indole compound(s) of formula (I) are used in proportions of between 0.2 and 3% by weight relative to the total weight of the composition (A).

17. Use according to any one of the preceding claims, characterized in that the compositions (A) and (B) are aqueous or consist of a mixture of water and a cosmetically acceptable solvent.

18. Use according to Claim 17, characterized in that the cosmetically acceptable solvent is chosen from C₁-C₄ lower alcohols, glycols, glycol ethers and lower esters.

19. Use according to any one of the preceding claims, characterized in that the composition (A) has a pH of between 5 and 10.

20. Use according to Claim 19, characterized in that the composition (A) has a pH of between 7 and 9.5.

21. Use according to any one of the preceding claims, characterized in that the composition (B) has a pH of between 5 and 11.

22. Use according to Claim 21, characterized in that the composition (B) has a pH of between 7 and 10.

23. Use according to any one of the preceding claims, characterized in that the compositions (A) and/or (B) are provided in the form of lotions which are thickened to a greater or lesser degree, gels, shampoos, emulsions such as milks and creams, or foams.

24. Use according to Claim 23, characterized in that the composition (A) is provided in the form of a gel and the composition (B) in the form of a shampoo.

25. Use according to any one of the preceding claims, characterized in that the compositions (A) and/or (B) may contain, in addition, at least one adjuvant chosen from anionic, cationic, non-ionic, amphoteric or zwitterionic surfactants or mixtures thereof, inorganic or organic thickening agents, antioxidants, penetrating agents, sequestrants, perfumes, buffers, dispersants, conditioners, film-forming agents, preservatives, opacifiers, sunscreens.

## Patentansprüche

1. Verwendung eines Metallsalzes in einem zweistufigen Verfahren zum Färben von Keratinfasern und insbesondere menschlichen Keratinfasern, wie dem Haar, wobei zu einem ersten Zeitpunkt eine Zusammensetzung (A) die in einem zum Färben geeigneten Medium mindestens eine Indolverbindung der folgenden Formel I enthält: 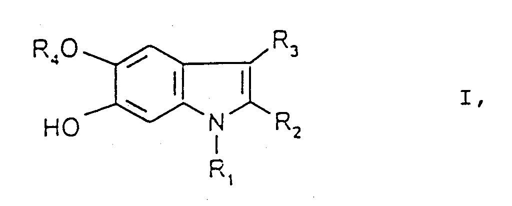 worin bedeuten:
R₁, R₂, R₃, und R₄, die gleich oder voneinander verschieden sind, Wasserstoff oder C₁₋₄-Alkyl, wobei R₁, R₂ und R₃ Wasserstoff bedeuten, wenn R₄ eine C₁₋₄-Alkylgruppe ist,
und zu einem zweiten Zeitpunkt eine Zusammensetzung (B) verwendet wird, die in einem zum Färben geeigneten Medium mindestens ein Metallsalz enthält, das unter Verbindungen ausgewählt ist, die eine katalytische Aktivität für die Oxidation mit Luftsauerstoff aufweisen und deren Redoxpotential gegenüber der Normalwasserstoffelektrode E₀ einen Wert von unter -0,25 V aufweist,
um Färbungen in Grautönen zu erhalten, die sich im Laufe der Zeit nicht verändern.

2. Verwendung nach Anspruch 1, dadurch gekennzeichnet, daß in Formel I die Alkylgruppe Methyl oder Ethyl ist und R₁, R₂ und R₃ nicht gleichzeitig eine C₁₋₄-Alkylgruppe bedeuten.

3. Verwendung nach einem der Ansprüche 1 oder 2, dadurch gekennzeichnet, daß die Indolverbindung(en) der Formel I unter 5,6-Dihydroxyindol, 2-Methyl-5,6-dihydroxyindol, 3-Methyl-5,6-dihydroxyindol, 1-Methyl-5,6-dihydroxyindol, 2,3-Dimethyl-5,6-dihydroxyindol und 5-Methoxy-6-hydroxyindol ausgewählt sind.

4. Verwendung nach Anspruch 3, dadurch gekennzeichnet, daß die Indolverbindung der Formel I 5,6-Dihydroxyindol ist.

5. Verwendung nach einem der vorhergehenden Ansprüche, dadurch gekennzeichnet, daß das Metallsalz oder die Metallsalze in gelöster oder dispergierter Form verwendet werden und unter Mangansalzen, Zinksalzen, Eisensalzen und/oder Cobaltsalzen sowie ihren Gemischen ausgewählt sind.

6. Verwendung nach Anspruch 5, dadurch gekennzeichnet, daß das Metallsalz oder die Metallsalze unter Acetaten, Halogeniden, Sulfaten und Carbonaten von Magnesium, Zink, Eisen und/oder Cobalt sowie den Hydraten dieser Verbindungen ausgewählt sind.

7. Verwendung nach Anspruch 6, dadurch gekennzeichnet, daß das Metallsalz oder die Metallsalze unter Mangandiacetattetrahydrat, Mangandichlorid und seinen Hydraten, Mangandihydrogencarbonat, Manganacetylacetonat, Mangantriacetat und seinen Hydraten, Mangantrichlorid, Zinkdichlorid, Zinkacetatdihydrat, Zinkcarbonat, Zinkdinitrat, Zinksulfat, Eisendichlorid, Eisensulfat, Eisendiacetat, Cobaltdiacetattetrahydrat, Cobaltcarbonat, Cobaltdichlorid, Cobaltdinitrat und Cobaltsulfatheptahydrat ausgewählt sind.

8. Verwendung nach Anspruch 7, dadurch gekennzeichnet, daß das Metallsalz oder die Metallsalze unter Mangandiacetattetrahydrat und Zinkdichlorid ausgewählt sind.

9. Verwendung nach einem der vorhergehenden Ansprüche, dadurch gekennzeichnet, daß das Metallsalz oder die Metallsalze in Anteilen von 0,001 bis 4 Gew.-%, ausgedrückt als Metall, bezogen auf das Gesamtgewicht der Zusammensetzung (B), verwendet werden.

10. Verwendung nach Anspruch 9, dadurch gekennzeichnet, daß das Metallsalz oder die Metallsalze in Anteilen von 0,005 bis 2 Gew.-%, ausgedrückt als Metall, bezogen auf das Gesamtgewicht der Zusammensetzung (B), verwendet werden.

11. Verwendung nach einem der vorhergehenden Ansprüche, dadurch gekennzeichnet, daß die Zusammensetzung (A) auf die Keratinfasern und insbesondere die menschlichen Keratinfasern, wie dem Haar, mit einer Einwirkungszeit von 15 s bis 30 min aufgebracht wird.

12. Verwendung nach einem der vorhergehenden Ansprüche, dadurch gekennzeichnet, daß die Zusammensetzung (B) auf die Keratinfasern und insbesondere die menschlichen Keratinfasern, wie dem Haar, mit einer Einwirkungszeit von 15 s bis 30 min aufgebracht wird.

13. Verwendung nach einem der vorhergehenden Ansprüche, dadurch gekennzeichnet, daß zwischen der Anwendung der Zusammensetzung (A) und der Zusammensetzung (B) ein Spülschritt liegt.

14. Verwendung nach einem der vorhergehenden Ansprüche, dadurch gekennzeichnet, daß die Indolverbindung(en) der Formel I in solchen Mengen werden, daß die Keratinfasern repigmentiert werden.

15. Verwendung nach Anspruch 14, dadurch gekennzeichnet, daß die Indolverbindung(en) der Formel I in Anteilen von 0,1 bis 5 Gew.-%, bezogen auf das Gesamtgewicht der Zusammensetzung (A), verwendet werden.

16. Verwendung nach Anspruch 15, dadurch gekennzeichnet, daß die Indolverbindung(en) der Formel I in Anteilen von 0,2 bis 3 Gew.-%, bezogen auf das Gesamtgewicht der Zusammensetzung (A), verwendet werden.

17. Verwendung nach einem der vorhergehenden Ansprüche, dadurch gekennzeichnet, daß die Zusammensetzungen (A) und (B) wäßrige Lösungen sind oder aus einem Gemisch aus Wasser und einem kosmetisch akzeptablen Lösungsmittel bestehen.

18. Verwendung nach Anspruch 17, dadurch gekennzeichnet, daß das kosmetisch akzeptable Lösungsmittel unter niederen C₁₋₄-Alkoholen, Glykolen, Glykolethern und niederen Estern ausgewählt ist.

19. Verwendung nach einem der vorhergehenden Ansprüche, dadurch gekennzeichnet, daß die Zusammensetzung (A) einen pH-Wert im Bereich von 5 bis 10 aufweist.

20. Verwendung nach Anspruch 19, dadurch gekennzeichnet, daß die Zusammensetzung (A) einen pH-Wert im Bereich von 7 bis 9,5 aufweist.

21. Verwendung nach einem der vorhergehenden Ansprüche, dadurch gekennzeichnet, daß die Zusammensetzung (B) einen pH-Wert im Bereich von 5 bis 11 aufweist.

22. Verwendung nach Anspruch 21, dadurch gekennzeichnet, daß die Zusammensetzung (B) einen pH-Wert im Bereich von 7 bis 10 aufweist.

23. Verwendung nach einem der vorhergehenden Ansprüche, dadurch gekennzeichnet, daß die Zusammensetzungen (A) und/oder (B) in Form von mehr oder weniger dickflüssiger Lotionen, als Gel, Haarwaschmittel, Emulsion, wie Milch, Creme oder Schaum vorliegen.

24. Verwendung nach Anspruch 23, dadurch gekennzeichnet, daß die Zusammensetzung (A) in Form eines Gels und die Zusammensetzung (B) als Haarwaschmittel vorliegt.

25. Verwendung nach einem der vorhergehenden Ansprüche, dadurch gekennzeichnet, daß die Zusammensetzungen (A) und/oder (B) ferner mindestens einen Hilfsstoff enthalten, der unter anionischen, kationischen, nichtionischen, amphoteren, zwitterionischen grenzflächenaktiven Mitteln oder ihren Gemischen, anorganischen oder organischen Verdickungsmitteln, Antioxidantien, Penetrationsmitteln, Maskierungsmitteln, Parfums, Puffern, Dispergiermitteln, Konditionierungsmitteln, Konservierungsmitteln, Trübungsmitteln und Sonnenschutzfiltern ausgewählt ist.
